# EUROPEAN PATENT APPLICATION

(11) **EP 4 219 680 A1**
(43) Date of publication of application: **02.08.2023**
(21) Application number: 21872876.4
(22) Date of filing: 17.09.2021
(51) Int. Cl.: C12N 1/20, A61K 35/741, A61P 27/02, A61P 25/00, A61P 29/00, A23L 33/135, C12R 1/25, C12R 1/01

(54) **NOVEL LACTIC ACID BACTERIA AND USE THEREOF**

(30) Priority: 23.09.2020 KR 20200123129
(71) Applicant: Pblbiolab, Seoul 02823 (KR); NVP Healthcare Co., Ltd., Suwon-si, Gyeonggi-do 16209 (KR)
(72) Inventor: KIM, Dong Hyun, Seoul 02823 (KR)
(74) Representative: Altmann Stößel Dick Patentanwälte PartG mbB
(86) International application number: PCT/KR2021/012888
(87) International publication number: WO 2022/065848

(57) **Abstract**

The present invention relates to novel *Lactobacillus plantarum, Bifidobacterium bifidum, Bifidobacterium longum* or mixtures thereof, and a use thereof. Strains or mixtures thereof according to the present invention have excellent *Proteobacteria* expression inhibitory and anti-inflammatory effects and increase lacrimal secretion from the eyes, inhibit inflammation in the cornea and/or retina, exhibit excellent anti-oxidant and inflammation inhibitory effects in the cornea and/or retina, reduce the amount of fatigue substances in the body, and exhibit effects such as neuroinflammatory factor inhibition, BDNF expression enhancement, and cognitive disorders and mental disorder alleviation. Therefore, the present invention can be effectively used in the treatment, alleviation and prevention of eye diseases, fatigue, inflammatory diseases, cognitive disorders, mental disorders and the like.

## Description

### [Technical Field]

The present invention relates to novel lactic acid bacteria of *Lactobacillus plantarum*, *Bifidobacterium bifidum*, and *Bifidobacterium longum*, or mixtures thereof.

### [Background Art]

In many cases of eye diseases related to aging, fatigue, etc., the eye diseases tend to develop not suddenly, but gradually depending on a person's age or other external factors such as fatigue. Diseases that are recognized to be detectable and treatable when a comprehensive eye examination is performed may include, for example, macular degeneration, cataract, glaucoma, diabetic retinopathy, etc. In a situation where the proportion of the elderly population is increasing and there is no choice but to receive a lot of visual stimuli that may cause eye fatigue, the prevalence and influence of the above-mentioned eye diseases are also increasing.

In the case of these eye diseases, there are major known problems of oxidizing major components of retinal pigment epithelial cells by the production of excessive reactive oxygen species (ROS) by oxidative stress and activating an intrinsic apoptosis pathway associated with mitochondrial dysfunction and DNA damage. Accordingly, various researches and developments are being conducted to treat diseases by targeting various signal pathways related to ROS. In addition, since inhibition of angiogenesis and accompanying inflammatory responses also stabilizes the blood wall to alleviate plasma leakage and retinal edema and increase the effect of treatment, the alleviation therefor is also required. In addition, recently, a relationship between these eye diseases and the imbalance of the intestinal microbiota has been confirmed through various literatures, and efforts to solve this problem through microorganisms have been partially confirmed.

Meanwhile, rather general and common diseases than the diseases may include eye fatigue and dry eye syndrome. Eye fatigue or dry eye syndrome is a disease in which symptoms such as blurry eyes, stiff eyes, foreign body sensation of eyes, severe dry eyes, eye stinging, sore eyes, eye strain, and eye pain are presented. Recently, the number of patients with eye diseases is rapidly increasing due to external factors such as polluted fine dust, long-time TV viewing, and excessive use of computers or smartphones.

In addition to the above-mentioned pathogenesis of most of the eye diseases including macular degeneration, glaucoma, eye fatigue, dry eye syndrome, etc. described above, it is known that the relation with inflammation should be considered very highly. It has been reported that inflammation-related cytokines such as TNF-α and IL-1β and inflammation-related chemokines such as IL-8 are overexpressed in the tears of patients with eye diseases than in normal people. It has been known that in NOD.B10.H2b mice with Sjogren's syndrome accompanied by eye diseases, the expression of IL-10 is low, and the intestinal microbiota of the feces corresponds to a state different from that of a normal group.

As a method for treating these eye diseases or alleviating eye fatigue, it is common to apply artificial tears/eye drops with similar ingredients to tears, and in severe cases, punctal occlusion, which closes the puncta with a punctal plug, has been used. In addition, commonly used therapeutic agents correspond to drugs known as immunosuppressants such as Cyclosporin A and Fluorometholone. A treatment method for the diseases may temporarily alleviate the diseases, but there are limitations in fundamentally treating the diseases, and there are many demands for new therapies.

Accordingly, the present inventors have completed the present invention by confirming novel strains that were effective in alleviation of eye diseases through functions such as anti-oxidant control, angiogenesis control, inflammation control, and lacrimal secretion control in the eyes in addition to alleviation effects on immune regulation, cognitive disorders, mental disorders, and inflammatory diseases.

### [Disclosure]

### [Technical Problem]

An object of the present invention is to provide *Lactobacillus plantarum* NK151 KCCM12783P.

Another object of the present invention is to provide *Bifidobacterium bifidum* NK175 KCCM12784P.

Another object of the present invention is to provide *Bifidobacterium longum* NK173 KCCM13046P.

Yet another object of the present invention is to provide a use for the prevention, alleviation or treatment of at least one selected from the group consisting of eye diseases, fatigue, cognitive disorders, mental disorders and inflammatory diseases, comprising *Lactobacillus plantarum* NK151 KCCM12783P, *Bifidobacterium bifidum* NK175 KCCM12784P, *Bifidobacterium longum* NK173 KCCM13046P or any mixture thereof.

### [Technical Solution]

One aspect of the present invention provides *Lactobacillus plantarum* NK151 (Depository Authority: Korean Culture Center of Microorganisms, Deposit Date: September 10, 2020. Accession Number: KCCM12783P).

The *Lactobacillus plantarum* NK151 KCCM12783P of the present invention may be a lactic acid bacterium isolated and identified from the intestinal microbiota (isolated from feces) of a healthy person.

A 16S rDNA base sequence for identification and classification of the *Lactobacillus plantarum* NK151 of the present invention is shown in SEQ ID NO: 1 attached hereto. Accordingly, the *Lactobacillus plantarum* NK151 of the present invention may include 16S rDNA represented by SEQ ID NO: 1.

As a result of analysis of the 16S rDNA base sequence represented by SEQ ID NO: 1, the *Lactobacillus plantarum* NK151 showed 99% homology with known strains of *Lactobacillus plantarum* to have the highest molecular phylogenetic relationship with *Lactobacillus plantarum.* Therefore, the lactic acid bacterium was identified as *Lactobacillus plantarum,* named as *Lactobacillus plantarum* NK151, and deposited with the Korean Culture Center of Microorganisms on September 10, 2020 (KCCM12783P).

The *Lactobacillus plantarum* NK151 of the present invention is a Gram-positive bacterium, and the cell type is a bacillus. More specific physiological characteristics of the *Lactobacillus plantarum* NK151 may be analyzed according to a conventional method in the art, and the results were shown in Table 4 below. Specifically, the *Lactobacillus plantarum* NK151 may use, as a carbon source, at least one selected from the group consisting of glycerol, L-arabinose, D-ribose, D-galactose, D-glucose, D-fructose, D-mannose, mannitol, α-methyl-D-mannoside, N-acetyl-glucosamine, amygdalin, arbutin, esculin, salicin, cellobiose, maltose, lactose, melibiose, sucrose, trehalose, melezitose, raffinose, gentiobiose, D-turanose and gluconate.

Another aspect of the present invention provides *Bifidobacterium bifidum* NK175 (Depository Authority: Korean Culture Center of Microorganisms, Deposit Date: September 10, 2020. Accession Number: KCCM12784P).

The *Bifidobacterium bifidum* NK175 KCCM12784P of the present invention may be a lactic acid bacterium isolated and identified from the intestinal microbiota (isolated from feces) of a healthy person.

A 16S rDNA base sequence for identification and classification of the *Bifidobacterium bifidum* NK175 KCCM12784P of the present invention is shown in SEQ ID NO: 2 attached hereto. Accordingly, the *Bifidobacterium bifidum* NK175 of the present invention may include 16S rDNA represented by SEQ ID NO: 2.

As a result of analysis of the 16S rDNA base sequence represented by SEQ ID NO: 2, the *Bifidobacterium bifidum* NK175 showed 99% homology with known strains of *Bifidobacterium bifidum* to have the highest molecular phylogenetic relationship with *Bifidobacterium bifidum.* Therefore, the lactic acid bacterium was identified as *Bifidobacterium bifidum*, named as *Bifidobacterium bifidum* NK175, and deposited with the Korean Culture Center of Microorganisms on September 10, 2020 (KCCM12784P).

The *Bifidobacterium bifidum* NK175 is a Gram-positive bacterium, and the cell type is a bacillus. More specific physiological characteristics of *Bifidobacterium bifidum* NK175 may be analyzed according to a conventional method in the art, and the results were shown in Table 5 below. Specifically, the *Bifidobacterium bifidum* NK175 may use, a carbon source, at least one selected from the group consisting of D-glucose, D-lactose, D-maltose, D-xylose, esculin, D-cellobiose, D-mannose and D-raffinose.

Yet another aspect of the present invention provides *Bifidobacterium longum* NK173 (Depository Authority: Korean Culture Center of Microorganisms, Deposit Date: September 6, 2021. Accession Number: KCCM13046P).

The *Bifidobacterium longum* NK173 KCCM13046P of the present invention may be a lactic acid bacterium isolated and identified from the intestinal microbiota (isolated from feces) of a healthy person.

A 16S rDNA base sequence for identification and classification of *Bifidobacterium longum* NK173 of the present invention is shown in SEQ ID NO: 3 attached hereto. Accordingly, the *Bifidobacterium longum* NK173 of the present invention may include 16S rDNA represented by SEQ ID NO: 3.

As a result of analysis of the 16S rDNA base sequence represented by SEQ ID NO: 3, the *Bifidobacterium longum* NK173 showed 99% homology with known strains of *Bifidobacterium longum* to have the highest molecular phylogenetic relationship with *Bifidobacterium longum.* Therefore, the lactic acid bacterium was identified as *Bifidobacterium longum* and named as *Bifidobacterium longum* NK173.

The *Bifidobacterium longum* NK173 of the present invention is a Gram-positive bacterium, and the cell type is a bacillus. More specific physiological characteristics of *Bifidobacterium longum* NK173 may be analyzed according to a conventional method in the art, and the results were shown in Table 6 below. Specifically, the *Bifidobacterium longum* NK173 may use, a carbon source, at least one selected from the group consisting of D-glucose, D-mannitol, D-maltose, D-xylose, L-arabinose, gelatin, esculin and D-mannose.

According to the present invention, the *Lactobacillus plantarum* NK151 KCCM12783P, *Bifidobacterium bifidum* NK175 KCCM12784P, *Bifidobacterium longum* NK173 KCCM13046P, or mixtures thereof have an excellent *Proteobacteria* expression inhibitory effect, and have characteristics of inhibiting the expression of inflammatory cytokines (e.g., TNF-α, etc.) and enhancing the expression of anti-inflammatory cytokines (e.g., IL-10, etc.). In addition, the *Lactobacillus plantarum* NK151 KCCM12783P, *Bifidobacterium bifidum* NK175 KCCM12784P, *Bifidobacterium longum* NK173 KCCM13046P, or mixtures thereof increase the lacrimal secretion from the eyes, inhibit inflammation in the cornea and/or retina, exhibit excellent anti-oxidant and inflammation inhibitory effects in the cornea and/or retina, reduce the amount of fatigue substances (e.g., plasma creatinine, lactic acid) in the body, and reduce the level of corticosterone. That is, the *Lactobacillus plantarum* NK151 KCCM12783P, *Bifidobacterium bifidum* NK175 KCCM12784P, *Bifidobacterium longum* NK173 KCCM13046P, or mixtures thereof may alleviate inflammation occurring in the eyes, effectively inhibit the level of nitric oxide in cells, minimize damage to the cornea, and enhance the eye health by preventing oxidative damage to the retina. In addition, the *Lactobacillus plantarum* NK151 KCCM12783P, *Bifidobacterium bifidum* NK175 KCCM12784P, *Bifidobacterium longum* NK173 KCCM13046P, or mixtures thereof exhibit an alleviation effect of inflammatory diseases including colitis, and exhibit effects such as neuroinflammatory factor inhibition, BDNF expression enhancement, and cognitive disorders and mental disorders alleviation.

Still another aspect of the present invention provides a pharmaceutical composition comprising *Lactobacillus plantarum* NK151 KCCM12783P, *Bifidobacterium bifidum* NK175 KCCM12784P, *Bifidobacterium longum* NK173 KCCM13046P, or any mixture thereof. In the present invention, the pharmaceutical composition may be a pharmaceutical composition comprising any one, two or three lactic acid bacteria described above.

Still yet another aspect of the present invention provides a pharmaceutical composition comprising *Lactobacillus plantarum* NK151 KCCM12783P, *Bifidobacterium bifidum* NK175 KCCM12784P, *Bifidobacterium longum* NK173 KCCM13046P, or any mixture thereof, and a pharmaceutically acceptable carrier.

Still yet another aspect of the present invention provides a pharmaceutical composition for preventing or treating eye diseases, inflammatory diseases, cognitive disorders or mental disorders, comprising *Lactobacillus plantarum* NK151 KCCM12783P, *Bifidobacterium bifidum* NK175 KCCM12784P, *Bifidobacterium longum* NK173 KCCM13046P, or any mixture thereof.

Specifically, the *Lactobacillus plantarum* NK 151 of the present invention may have an effect of preventing, treating or alleviating at least one disease selected from the group consisting of eye diseases, inflammatory diseases, cognitive disorders and mental disorders. In addition, specifically, the *Bifidobacterium bifidum* NK175 of the present invention may have an effect of preventing, treating or alleviating at least one disease selected from the group consisting of eye diseases, inflammatory diseases, cognitive disorders and mental disorders. In addition, specifically, the *Bifidobacterium longum* NK173 of the present invention may have an effect of preventing, treating or alleviating at least one disease selected from the group consisting of eye diseases, inflammatory diseases, cognitive disorders and mental disorders. In addition, the mixtures in any mixed form of the above-mentioned strains may have an effect of preventing, treating, or alleviating at least one disease selected from the group consisting of eye diseases, inflammatory diseases, cognitive disorders and mental disorders.

Still yet another aspect of the present invention provides a pharmaceutical composition for preventing or treating eye diseases, comprising *Lactobacillus plantarum* NK151 KCCM12783P, *Bifidobacterium bifidum* NK175 KCCM12784P, *Bifidobacterium longum* NK173 KCCM13046P, or any mixture thereof.

Still yet another aspect of the present invention provides a pharmaceutical composition for preventing or treating inflammatory diseases, comprising *Lactobacillus plantarum* NK151 KCCM12783P, *Bifidobacterium bifidum* NK175 KCCM12784P, *Bifidobacterium longum* NK173 KCCM13046P, or any mixture thereof.

Still yet another aspect of the present invention provides a pharmaceutical composition for preventing or treating cognitive disorders, comprising *Lactobacillus plantarum* NK151 KCCM12783P, *Bifidobacterium bifidum* NK175 KCCM12784P, *Bifidobacterium longum* NK173 KCCM13046P, or any mixture thereof.

Still yet another aspect of the present invention provides a pharmaceutical composition for preventing or treating mental disorders, comprising *Lactobacillus plantarum* NK151 KCCM12783P, *Bifidobacterium bifidum* NK175 KCCM12784P, *Bifidobacterium longum* NK173 KCCM13046P, or any mixture thereof.

In the present invention, the "eye diseases" are diseases occurring in the eyes, and include diseases occurring in the structures of the eye by including abnormalities of the conjunctiva and cornea, which are structures covering the front of the eye, and abnormalities of the anterior part of the eyeball and the anterior structures including the iris or lens.

Examples of these eye diseases include, for example, eye fatigue, retinopathy, keratitis, macular degeneration (including dry and/or wet), dry eye syndrome, retinitis pigmentosa, diabetic retinopathy, retinopathy of prematurity, proliferative retinopathy, ischemic retinopathy, choroidal neovascularization, neovascular glaucoma, ischemic optic neuropathy, diabetic macular degeneration, erythema, myopia, Sjogren's syndrome, etc., but are not limited thereto. Preferably, the eye diseases may be at least one selected from the group consisting of eye fatigue, macular degeneration, dry eye syndrome, retinopathy, and keratitis.

The "eye fatigue" refers to fatigue in the eyes caused by applying the overload to the ciliary muscle due to control loss due to presbyopia during near-distance work, drying eyeballs due to a dry environment, and exposing excessive light. Symptoms such as blurring, tears, congestion, glare, stiffness, and foreign body sensation appear, and in severe cases, pain and headaches may occur. Such eye fatigue may be caused, for example, when the tear film is not maintained, the ocular surface becomes dry, and as a result, scars occur on the cornea and conjunctiva, and may cause direct damage to retinal cells and lens in the eyeball when free radicals are generated in retinal epithelial cells by excessive light stimulation.

The "macular degeneration" refers to progressive damage to the macula, which is close to the center of the retina and is responsible for detailed vision. The macular degeneration includes dry macular degeneration, such as when retinal lesions such as drusen (a state in which waste products accumulate in the macula) or retinal pigment epithelium atrophy, wet macular degeneration caused by the growth of choroidal neovascularization under the retina, and the like.

The "dry eye syndrome" includes blurred eyes, stiff eyes, foreign body sensation in the eyes, eye fatigue, severe dry eyes, stinging eyes, sore eyes, eye strain, eye pain, headache caused by eye redness and dry eye, decreased sensitivity of the corneal nerve, various inflammations, and the like, which are generated due to dry eye caused by lack of tears, or excessive evaporation of tears.

The "retinopathy" refers to a case of causing continuous or severe damage to the retina of the eye, and includes diseases caused by circulatory failure in peripheral blood vessels of the retina or by accumulation of deposits thereon. The retinopathy includes diabetic retinopathy, hypertensive retinopathy, retinopathy of prematurity, radiation retinopathy, and the like.

The "conjunctivitis" refers to an eye disease in which the conjunctiva is congested due to inflammation of the conjunctiva, causing eye mucus, follicles are formed on the inner side of the eyelid, and itching and foreign body sensation are felt.

In the present invention, the inflammatory disease is a general term for diseases in which inflammation is a main lesion, and may be at least one selected from the group consisting of inflammatory bowel disease, arthritis, gout, hepatitis, obesity, gastritis, nephritis, diabetes, tuberculosis, bronchitis, pleurisy, peritonitis, spondylitis, pancreatitis, urethritis, cystitis, vaginitis, arteriosclerosis, sepsis and periodontitis.

The term of inflammatory bowel disease (IBD) refers to a class of inflammatory conditions of the colon and gastrointestinal tract. The main types of IBD are ulcerative colitis (UC) and Crohn's disease. A key difference between UC and Crohn's disease is the location and properties of the inflammatory changes. Crohn's disease may affect any part of the gastrointestinal tract from the mouth to the anus, whereas the UC is limited to the colon and rectum. A definitive diagnosis of Crohn's disease or UC may not be made due to the characteristic of the presentation. In such cases, indeterminate colitis diagnosis may be made. Other types of IBD include collagenous colitis, lymphocytic colitis, ischemic colitis, diversion colitis, Behcet's disease, and indeterminate colitis, but are not limited thereto.

That is, the IBD according to the present invention may be at least one selected from the group consisting of ulcerative colitis, Crohn's disease, collagenous colitis, lymphocytic colitis, ischemic colitis, diversion colitis, Behcet's disease, and indeterminate colitis.

In the present invention, the cognitive disorders refer to disorders that exhibit cognitive impairment and behavioral changes, and refer to diseases caused by malfunctions of memory, spatial perception, judgment, executive function, and language ability. The cognitive disorders may be at least one selected from the group consisting of, for example, Alzheimer's disease, Huntington's disease, vascular dementia, Pick's disease, Parkinson's disease, Creutzfeldt-Jakob disease, and dementia, but are not limited thereto. In addition, the cognitive disorders include memory impairment, symptoms of dementia, and the like caused from the symptoms.

In the present invention, the "mental disorders" are also referred to as mental diseases or mental illnesses, and refer to behavioral-mental abnormalities that cause problems in personal and social functions, and may include physical symptoms caused by the behavioral-mental abnormalities. The causes of the mental disorders may include congenital brain problems and serious stress factors, and the like. Types of mental disorders may be at least one selected from the group consisting of depression anxiety disorder, mood disorder, insomnia, delusional disorder, obsessive-compulsive disorder, migraine, stress, memory disorder, autism, attention-deficit hyperactivity disorder (ADHD), attention-deficit disease (ADD), panic attack and attention disorder, but are not limited thereto.

In the present invention, the "neuroinflammation" refers to inflammation occurring in the brain, and is an important factor in causing diseases related to cognitive disorders and mental disorders. It has been known that when inflammatory cells are excessively activated in the brain, the secretion of pro-inflammatory cytokines is increased, and cognitive disorders or mental disorders are caused by overactivation of these brain inflammatory reactions according to brain cell damage.

According to an embodiment of the present invention, it was confirmed that *Lactobacillus plantarum* NK151, *Bifidobacterium bifidum* NK175 and/or *Bifidobacterium longum* NK173 exhibit excellent anti-inflammatory effects by inhibiting the expression of TNF-α and IL-1β of macrophages while inducing the expression of IL-10.

In addition, according to an embodiment of the present invention, it was confirmed that *Lactobacillus plantarum* NK151, *Bifidobacterium bifidum* NK175 and/or *Bifidobacterium longum* NK173 exhibit an effect of achieving normalization of intestinal microbiota by inhibiting *Proteobacteria* growth activity.

In addition, according to an embodiment of the present invention, it was confirmed that *Lactobacillus plantarum* NK151, *Bifidobacterium bifidum* NK175 and/or *Bifidobacterium longum* NK173 restore the lacrimal secretion in an eye disease animal model, inhibit corneal inflammation, control the expression levels of inflammatory cytokines and anti-inflammatory cytokines, control the levels of malondialdehyde (MDA), glutathione (GSH) and nitric oxide (NO) in the retina, and reduce the levels of lactic acid, lactate dehydrogenase, creatinine and corticosterone, which are fatigue substances (especially, highly related to eye fatigue) in the blood.

In addition, according to an embodiment of the present invention, it was confirmed that *Lactobacillus plantarum* NK151, *Bifidobacterium bifidum* NK175 and/or *Bifidobacterium longum* NK173 inhibit a decrease in colon length in a colitis animal model, reduce MPO expression, reduce the expression of inflammatory cytokines in the colon, and increase the expression level of anti-inflammatory cytokines.

In addition, according to an embodiment of the present invention, it was confirmed that *Lactobacillus plantarum* NK151, *Bifidobacterium bifidum* NK175 and/or *Bifidobacterium longum* NK173 alleviate behavioral problems caused by the diseases in animal models with dementia, anxiety and depression (alteration alleviation), regulate neuroinflammation by reducing the expression of inflammatory cytokines, increase the expression of BDNF, and exhibit the alleviation and treatment effects on the diseases through the alleviation of anxiety/depression symptoms (reduction of immobility time in a suspended tail state).

The results indicate that *Lactobacillus plantarum* NK151, *Bifidobacterium bifidum* NK175 and/or *Bifidobacterium longum* NK173 exhibit excellent efficacies on the prevention, treatment or alleviation of inflammatory diseases, eye diseases, cognitive disorders, and/or mental disorders.

In addition, the pharmaceutical composition normalizes the changed intestinal microbiota and simultaneously normalizes the bowel immunity to exhibit excellent effects of controlling, preventing, alleviating and treating eye diseases, cognitive disorders, mental disorders, inflammatory diseases, and complications caused thereby.

The strains according to the present invention may be used in various forms such as viable cells, dead cells, cultures, lysates or extracts. Even if the strains according to the present invention have any form under the condition including the strains, the strains show results of equivalent level or higher in terms of the above-mentioned effects (especially, considering live bacteria).

The viable cells refer to live bacteria as they are, and the dead cells mean that effective ingredients are isolated and extracted by methods such as heat drying, pressurization and drug treatment after culturing viable cells and the like under certain conditions.

The culture refers to an object obtained by culturing lactic acid bacteria in a known liquid medium or solid medium, and is a concept including the strains according to the present invention. The product may include lactic acid bacteria. The medium may be selected from known liquid media or solid media, and may be, for example, an MRS liquid medium, a GAM liquid medium, an MRS agar medium, a GAM agar medium, and a BL agar medium, but is not limited thereto.

The lysate means to have a crushed form by isolating and processing viable cells, dead cells or cultures thereof through mechanical and chemical methods. For example, the crushed form may be prepared through bead mills, presses, sonicators or microfluidizers, enzymatic treatment, and the like.

The extract means extracting viable cells, dead cells and/or lysates with known extraction methods (obtained by extraction with known extract solvents (e.g., water, C1 to C4 alcohols (methanol, ethanol, etc.)).

In the present invention, it has been confirmed that mixtures obtained by mixing the strains exhibit a synergistic effect, which include the use form of any mixture thereof.

The combined form or the used form of the mixture may include, for example, forms of
1) *Lactobacillus plantarum* NK151 and *Bifidobacterium bifidum* NK175;
2) *Lactobacillus plantarum* NK151 and *Bifidobacterium longum* NK173; or
3) *Lactobacillus plantarum* NK151, *Bifidobacterium bifidum* NK175 and *Bifidobacterium longum* NK173.

That is, an excellent synergistic effect may be exhibited by mixing *Bifidobacterium* sp. strains with the *Lactobacillus plantarum* NK151 according to the present invention.

The ratio of *Lactobacillus plantarum* NK151 and *Bifidobacterium bifidum* NK175 may be, for example, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9 or 1 : 10, based on colony forming units (CFU). The ratio of *Lactobacillus plantarum* NK151 and *Bifidobacterium bifidum* NK175 may be used in preferably 9 : 1 to 1 : 1, more preferably 4 : 1 to 1 : 1, and more specifically 4:1.

The ratio of *Lactobacillus plantarum* NK151 and *Bifidobacterium longum* NK173 may be, for example, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9 or 1 : 10, based on colony forming units (CFU). The ratio of *Lactobacillus plantarum* NK151 and *Bifidobacterium longum* NK173 may be used in preferably 9 : 1 to 1 : 1, more preferably 4 : 1 to 1 : 1, and more specifically 4 : , based on colony forming units (CFU).

The *Lactobacillus plantarum* NK151, *Bifidobacterium bifidum* NK175 and *Bifidobacterium longum* NK173 may consider the CFU based on a sum of two types of *Bifidobacterium* sp. strains with *Lactobacillus plantarum* NK151. For example, based on colony forming units (CFU), the ratio of *Lactobacillus plantarum* NK151 to *Bifidobacterium bifidum* NK175 and *Bifidobacterium longum* NK173 may be 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9 or 1 : 10. The ratio of *Lactobacillus plantarum* NK151 to *Bifidobacterium bifidum* NK175 and *Bifidobacterium longum* NK173 may be used in preferably 9 : 1 to 1 : 1, more preferably 4 : 1 to 1 : 1, and more specifically 4:1.

According to an embodiment of the present invention, the mixture of the strains, that is, the combined form, shows an excellent synergistic effect to increase the lacrimal secretion in an eye disease animal model, alleviate corneal damage, control the expression of inflammatory cytokines and chemokines, and exhibited excellent effects in regulating anti-oxidant in the retina and controlling eye fatigue.

According to an embodiment of the present invention, the mixture of the strains also exhibits excellent effects on treatment and alleviation of diseases through a synergistic effect by strain mixing even for inflammatory diseases (in particular, inflammatory bowel disease), cognitive disorders and mental disorders.

In particular, in terms of normalization of intestinal microbiota, the mixing of the strains may exhibit a better effect in maintaining intestinal health compared to a single strain.

The pharmaceutical composition of the present invention may be prepared in pharmaceutical formulations by using methods known in the art so as to provide rapid, sustained, or delayed release of the active ingredient after being administrated to mammals. In preparing the formulations, the pharmaceutical composition according to the present invention may additionally include a pharmaceutically acceptable carrier within a range without inhibiting the activity of the compound of the present invention.

The pharmaceutically acceptable carrier may include commonly used carriers, for example, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil, but is not limited thereto. In addition, the pharmaceutical composition of the present invention may include diluents or excipients such as fillers, extenders, binders, wetting agents, disintegrants, surfactants, and other pharmaceutically acceptable additives.

The pharmaceutical composition according to the present invention may be administered in a pharmaceutically effective amount. The "pharmaceutically effective amount" means an amount enough to prevent or treat diseases with a reasonable benefit/risk ratio applicable to medical treatment. The effective dosage level may be variously selected by those skilled in the art depending on factors such as a formulation method, the condition and weight of a patient, the sex and age of a patient, disease severity, drug form, administration route and duration, excretion rate, reaction sensitivity, and the like. The effective amount may vary depending on a route of treatment, the use of excipients and a possibility of use with other agents, as appreciated by those skilled in the art. However, for a desirable effect, in the case of oral administration, the composition of the present invention may be administered to adults at 0.0001 to 100 mg/kg per day, preferably 0.001 to 100 mg/kg per 1 kg of body weight at a day, but the dosage is not intended to limit the scope of the present invention in any way.

The pharmaceutical composition of the present invention may be administered to mammals such as mice, livestock, and humans through various routes. Specifically, the pharmaceutical composition of the present invention may be administered orally or parenterally (e.g., coated or injected intravenously, subcutaneously, or intraperitoneally), but preferably administered orally. Solid preparations for oral administration may include powders, granules, tablets, capsules, soft capsules, pills, and the like. Liquid preparations for oral administration may correspond to suspensions, oral liquids, emulsions, syrups, aerosols and the like, and may include various excipients, for example, a wetting agent, a sweetener, an aromatic agent, a preservative, and the like, in addition to water and liquid paraffin which are commonly used as simple diluents. Preparations for parenteral administration may be formulated and used in the forms of external preparations such as sterilized aqueous solutions, liquids, non-aqueous solvents, suspensions, emulsions, eye drops, eye ointments, syrups, suppositories, aerosols, etc., and sterile injectable preparations according to a general method, respectively. Preferably, a pharmaceutical composition of creams, gels, patches, sprays, ointments, plasters, lotions, liniment agents, eye ointments, eye drops, pasta agents, or cataplasmas may be prepared and used, but is not limited thereto. Preparations for topical administration may be in anhydrous or aqueous form depending on a clinical prescription. As the non-aqueous solution and the suspension, propylene glycol, polyethylene glycol, vegetable oil such as olive oil, injectable ester such as ethyl oleate, and the like may be used. As a base of the suppository, witepsol, macrogol, tween 61, cacao butter, laurinum, glycerogelatin, and the like may be used.

Yet another aspect of the present invention provides a method for the prevention or treatment of at least one disease selected from the group consisting of eye diseases, cognitive disorders, mental disorders and inflammatory diseases, comprising *Lactobacillus plantarum* NK151, *Bifidobacterium bifidum* NK175, *Bifidobacterium longum* NK173 or any mixture thereof.

The "*Lactobacillus plantarum* NK151", "*Bifidobacterium bifidum* NK175", "*Bifidobacterium longum* NK173", "eye diseases", "cognitive disorders", "mental disorders" and "inflammatory diseases" are the same as described above.

The pharmaceutical composition of the present invention may be administered as an individual therapeutic agent or in combination with other therapeutic agents, and sequentially or simultaneously administered with conventional therapeutic agents. In addition, the pharmaceutical composition of the present invention may be administered singly or multiply. It is important to administer an amount capable of obtaining a maximum effect with a minimal amount without side effects in consideration with all the factors, and the amount thereof may be easily determined by those skilled in the art.

The term "subject" used herein includes animals or humans of which symptoms may be improved by administration of the pharmaceutical composition according to the present invention. By administering the therapeutic composition according to the present invention to the subject, it is possible to effectively prevent and treat eye diseases, cognitive disorders, mental disorders, or inflammatory diseases.

The term "administration" of the present invention means introducing a predetermined substance to humans or animals by any suitable method. The administration route of the therapeutic composition according to the present invention may be administered orally or parenterally through any general route as long as reaching a target tissue. In addition, the therapeutic composition of the present invention may be administered by any device capable of transferring an active ingredient to target cells.

A preferable dose of the pharmaceutical composition of the present invention varies according to the condition and body weight of a patient, the severity of a disease, a drug form, and the route and period of administration, but may be properly selected by those skilled in the art.

The present invention provides *Lactobacillus plantarum* NK151, *Bifidobacterium bifidum* NK175, *Bifidobacterium longum* NK173 or any mixture thereof for use in prevention or treatment of at least one disease selected from the group consisting of eye diseases, cognitive disorders, mental disorders and inflammatory diseases.

The present invention relates to *Lactobacillus plantarum* NK151, *Bifidobacterium bifidum* NK175, *Bifidobacterium longum* NK173 or any mixture thereof for use in the prevention or treatment of inflammatory diseases.

The present invention relates to *Lactobacillus plantarum* NK151, *Bifidobacterium bifidum* NK175, *Bifidobacterium longum* NK173 or any mixture thereof for use in the prevention or treatment of eye diseases.

The present invention relates to *Lactobacillus plantarum* NK151, *Bifidobacterium bifidum* NK175, *Bifidobacterium longum* NK173 or any mixture thereof for use in the prevention or treatment of cognitive disorders.

The present invention relates to *Lactobacillus plantarum* NK151, *Bifidobacterium bifidum* NK175, *Bifidobacterium longum* NK173 or any mixture thereof for use in the prevention or treatment of mental disorders.

The present invention provides a use of *Lactobacillus plantarum* NK151, *Bifidobacterium bifidum* NK175, *Bifidobacterium longum* NK173 or any mixture thereof in preparation of a medicament for the treatment of at least one disease selected from the group consisting of eye diseases, cognitive disorders, mental disorders and inflammatory diseases.

The present invention relates to a use of *Lactobacillus plantarum* NK151, *Bifidobacterium bifidum* NK175, *Bifidobacterium longum* NK173 or any mixture thereof in preparation of a medicament for the treatment of inflammatory diseases.

The present invention relates to a use of *Lactobacillus plantarum* NK151, *Bifidobacterium bifidum* NK175, *Bifidobacterium longum* NK173 or any mixture thereof in preparation of a medicament for the treatment of eye diseases.

The present invention relates to a use of *Lactobacillus plantarum* NK151, *Bifidobacterium bifidum* NK175, *Bifidobacterium longum* NK173 or any mixture thereof in preparation of a medicament for the treatment of cognitive disorders.

The present invention relates to a use of *Lactobacillus plantarum* NK151, *Bifidobacterium bifidum* NK175, *Bifidobacterium longum* NK173 or any mixture thereof in preparation of a medicament for the treatment of mental disorders.

Yet another aspect of the present invention provides a food composition for the prevention or alleviation of at least one disease selected from the group consisting of eye diseases, cognitive disorders, mental disorders and inflammatory diseases, comprising *Lactobacillus plantarum* NK151, *Bifidobacterium bifidum* NK175, *Bifidobacterium longum* NK173 or any mixture thereof.

Yet another aspect of the present invention relates to a food composition for the alleviation of fatigue, comprising *Lactobacillus plantarum* NK151, *Bifidobacterium bifidum* NK175, *Bifidobacterium longum* NK173, or any mixture thereof.

The food composition according to the present invention exhibits an excellent effect on the alleviation of fatigue by reducing the level of stress or fatigue substances in the body. Specifically, the food composition may exhibit excellent effects on the alleviation of fatigue by reducing the levels of lactic acid, lactate dehydrogenase, creatinine and corticosterone, and enhancing an anti-oxidant effect.

The "*Lactobacillus plantarum* NK151", "*Bifidobacterium bifidum* NK175", "*Bifidobacterium longum* NK173", "eye diseases", "cognitive disorders", "mental disorders" and "inflammatory diseases" are the same as described above.

Specifically, the lactic acid bacteria included in the food composition of the present invention may be viable cells thereof, dead cells thereof, cultures thereof, lysates thereof or extracts thereof, but may be used without limitation as long as a form of lactic acid bacteria may achieve the effect of preventing or alleviating cognitive disorders, eye diseases, mental disorders or inflammatory diseases.

The type of food is not particularly limited. Foods which may be added with the lactic acid bacteria include sausages, meat, bread, chocolates, snacks, candies, confectionery, ramen, pizza, other noodles, gums, dairy products including ice cream, various soups, beverages, tea, drinks, alcohol drinks, vitamin complexes, and the like. When the food is formulated into beverages, liquid ingredients added in addition to the novel lactic acid bacteria are not limited thereto, but may contain various flavoring agents or natural carbohydrates as additional ingredients, as in conventional beverages. The natural carbohydrates described above may be monosaccharides (e.g., glucose, fructose, etc.), disaccharides (e.g., maltose, sucrose, etc.), polysaccharides (e.g., general sugars such as dextrin, cyclodextrin, etc.), and sugar alcohols such as xylitol, sorbitol, erythritol, etc.

The type of food may specifically be a health functional food. The health functional food may contain various nutrients, vitamins, minerals (electrolytes), flavoring agents such as synthetic and natural flavoring agents, coloring agents and enhancers (cheese, chocolate, etc.), pectic acid and salts thereof, organic acid, a protective colloidal thickener, a pH adjusting agent, a stabilizer, a preservative, glycerin, alcohol, a carbonic acid agent used in a carbonated drink, and the like. These ingredients may be used alone or in combination, and the ratio of these additives may be generally selected in the range of 0.001 to 50 parts by weight per total weight of the composition.

The health functional food is a food that emphasizes a biomodulating function of food, and is a food that gives an added value to be applied and presented for a specific purpose by using physical, biochemical, and bioengineering methods. These ingredients of the health functional food are designed and processed to fully exert the biomodulating functions associated with biodefense, regulation of biorhythm, prevention and recovery of diseases to a living body, and may contain food acceptable food supplement additives, sweeteners or functional raw materials.

When *Lactobacillus plantarum* NK151, *Bifidobacterium bifidum* NK175 and/or *Bifidobacterium longum* NK173 of the present invention are used as a health functional food (or health functional beverage additive), the *Lactobacillus plantarum* NK151, *Bifidobacterium bifidum* NK175 and/or *Bifidobacterium longum* NK173 are added as they are or used together with other foods or food ingredients, and may be used suitably according to conventional methods. The mixed amount of the *Lactobacillus plantarum* NK151, *Bifidobacterium bifidum* NK175 and/or *Bifidobacterium longum* NK173 may be suitably determined according to use purposes thereof (prevention, health or alleviation, therapeutic treatment).

### [Advantageous Effects]

According to the present invention, the novel lactic acid bacteria and any mixture thereof have an excellent *Proteobacteria* expression inhibitory effect, and have characteristics of inhibiting the expression of inflammatory cytokines (e.g., TNF-α, etc.) and enhancing the expression of anti-inflammatory cytokines (e.g., IL-10, etc.). In addition, the novel lactic acid bacteria and any mixture thereof increase the lacrimal secretion from the eyes, inhibit inflammation in the cornea and/or retina, exhibit excellent anti-oxidant and inflammation inhibitory effects in the cornea and/or retina, reduce the amount of fatigue substances (e.g., plasma creatinine, lactic acid, etc.) in the body, and reduce the level of corticosterone. In addition, the novel lactic acid bacteria and any mixture thereof exhibit an alleviation effect of inflammatory diseases including colitis, and exhibit effects such as neuroinflammatory factor inhibition, BDNF expression enhancement, and cognitive disorders and mental disorders alleviation. Therefore, the novel lactic acid bacteria and any mixture thereof may be usefully used for treatment, alleviation and prevention of eye diseases, inflammatory diseases, cognitive disorders, mental disorders, and the like.

It should be understood that the effects of the present invention are not limited to the effects described above, but include all effects that can be deduced from the detailed description of the present invention or configurations of the invention described in claims.

### [Modes of the Invention]

Hereinafter, the present invention will be described in detail by Examples. However, the following Examples are just illustrative of the present invention, and the present invention is not limited to the following Examples.

### Example 1. Isolation and identification of lactic acid bacteria of Lactobacillus plantarum NK151, Bifidobacterium longum NK173 and Bifidobacterium bifidum NK175

### 1-1. Isolation of lactic acid bacteria from human feces

In order to obtain a novel strain from the intestinal microbiota of a healthy person, human feces were added and suspended in a GAM broth (Nissui Pharmaceutical, Japan). Thereafter, a supernatant was taken and transplanted to a general anaerobic medium (GAM) agar medium (Nissui Pharmaceutical, Japan) and a BL agar medium (Nissui Pharmaceutical, Japan), and incubated at 37°C for about 48 hours, and then *Lactobacillus* sp. and *Bifidobacterium* sp. strains forming colonies were isolated.

### 1-2. Identification of isolated lactic acid bacteria

In order to confirm the physiological characteristics of the strains isolated from the human feces and the phylogenetic positions of the selected strains, by analyzing a base sequence of 16S rDNA, the species of the strains were determined and the strain names were assigned.

The strains isolated through the procedure were shown in Table 1 below.

**[Table 1]**

| No. | Strain name | No. | Strain name |
|---|---|---|---|
| 1 | *Lactobacillus plantarum* NK151 | 16 | *Lactobacillus reuteri* NK 166 |
| 2 | *Lactobacillus plantarum* NK 152 | 17 | *Lactobacillus johnsonii* NK 167 |
| 3 | *Lactobacillus casei* NK 153 | 18 | *Lactobacillus johnsonii* NK 168 |
| 4 | *Lactobacillus casei* NK154 | 19 | *Lactobacillus rhamnosus* NK169 |
| 5 | *Lactobacillus gasseri* NK155 | 20 | *Lactobacillus rhamnosus* NK 170 |
| 6 | *Lactobacillus gasseri* K156 | 21 | *Bifidobacterium adolescentis* NK 171 |
| 7 | *Lactobacillus salivarius* NK 157 | 22 | *Bifidobacterium adolescentis* NK172 |
| 8 | *Lactobacillus sakei* NK158 | 23 | *Bifidobacterium longum* NK 173 |
| 9 | *Lactobacillus paracasei* NK159 | 24 | *Bifidobacterium longum* NK 174 |
| 10 | *Lactobacillus helveticus* NK160 | 25 | *Bifidobacterium bifidum* NK 175 |
| 11 | *Lactobacillus bulgaricus* NK161 | 26 | *Bifidobacterium bifidum* NK 176 |
| 12 | *Lactobacillus pentosus* NK 162 | 27 | *Bifidobacterium breve* NK177 |
| 13 | *Lactobacillus brevis* NK 163 | 28 | *Bifidobacterium breve* NK 178 |
| 14 | *Lactobacillus brevis* NK164 | 29 | *Bifidobacterium animalis* NK 179 |
| 15 | *Lactobacillus reuteri* NK 165 | 30 | *Bifidobacterium animalis* NK 180 |

### Example 2. Characteristic analysis of strains

### 2-1. Measurement of expression levels of IL-10 and TNF-α in macrophages

2 ml of sterile 4% thioglycolate was administered intraperitoneally to 6-week-old male C57BL/6 mice (20 to 23 g), the mice were anesthetized after 96 hours, 8 ml of an RPMI 1640 medium was administered to the abdominal cavity of the mouse, and after 5 to 10 minutes, the RPMI medium (macrophages) was isolated from the abdominal cavity of the mouse, centrifuged at 1000 g for 10 minutes, and washed twice with the RPMI 1640 medium. The macrophages were spread on a 24-well plate at the number of 0.5 × 10⁶ per well, and only the lactic acid bacteria identified in Example 1 were treated or both lactic acid bacteria and an inflammatory response inducer LPS were treated for 24 hours, and then the expression levels of TNF-α and IL-10 cytokines in the supernatant were measured using an ELISA kit (Pierce Biotechology, Inc., Rockford, IL, USA).

First, 50 µl of the supernatant was put into a 96-well plate coated with TNF-α and IL-10 antibodies, respectively, reacted at room temperature for 2 hours, and then washed with phosphate buffered saline-Tween (PBS-Tween), and added with a color developing agent and developed for 20 minutes, and the absorbance was measured and calculated at a wavelength of 450 nm.

### 2-2 Proteobacteria growth inhibitory activity

*Proteobacteria* growth inhibitory activity was measured. 1 × 10⁶ CFU/mL of a human fecal dilution and 1 × 10⁶ CFU/mL of a lactic acid bacteria culture dilution isolated in Example 1 were inoculated by 0.1 mL each into 10 mL of GAM medium, incubated anaerobically for 24 hours, and collected, and then DNA was extracted with a QIAamp DNA stool mini kit (Qiagen, Germany), and qPCR was performed. The PCR reaction was first treated at 95°C for 30 seconds, and then repeatedly reacted 42 times at 95°C for 5 seconds and at 72°C for 30 seconds.
qPCR primers were shown in Table 2 below.

**[Table 2]**

| SEQ ID NO: | Primer type | Sequence |
|---|---|---|
| 4 | Proteobacteria_Forward | 5'- GCTAACGCATTAAGTRYCC CG-3' |
| 5 | Proteobacteria_Reverse | 5’-GCC ATGCRGCACCTGTCT-3’ |
| 6 | Bacteria_Forward | 5’- TCGTCGGCAGCGTCAGATG TGTATAAGAGACAGGTGCC AGCMGCCGCGGTAA-3’ |
| 7 | Bacteria_Reverse | 5’- GTCTCGTGGGCTCGGAGAT GTGTATAAGAGACAGGGA CTACHV GGGTWTCTAAT-3’ |

### 2-3 Confirmation of characteristic analysis results

As shown in Table 3, *Proteobacteria* growth inhibitory activity and expression changes of inflammatory cytokines and anti-inflammatory cytokines in macrophages were confirmed.

**[Table 3]**

| No. | Strain name | *Proteob acteria* growth inhibiti on | LPS-untreated macrophages | | | LPS-treated macrophages | | |
|---|---|---|---|---|---|---|---|---|
| | | | IL-10 expressio n level ([Lactic acid bacteria treated group/co ntrol group] × 100) (A) | TNF-α expressio n level ([Lactic acid bacteria treated group/co ntrol group] × 100) (B) | (A/B ) × 100 | IL-10 expressi on level ([Lactic acid bacteria treated group/co ntrol group] × 100) (C) | TNF-α expressi on level ([Lactic acid bacteria treated group/co ntrol group] × 100) (D) | (C D) |
| 1 | *Lactob acillus plantar um* NK151 | ++ | +++ | + | ++ | + | - | + + + |
| 2 | *Lactob acillus plantar um* NK152 | + | + | + | + | - | - | + |
| 3 | *Lactob acillus casei* NK153 | + | + | ++ | - | + | - | + |
| 4 | *Lactob acillus casei* NK154 | + | + | + | - | + | + | + |
| 5 | *Lactob acillus gasseri* NK155 | + | + | ++ | - | + | - | + + |
| 6 | *Lactob acillus gasseri* K156 | + | + | + | - | + | + | + |
| 7 | *Lactob acillus salivari us* NK157 | + | + | + | + | + | - | + |
| 8 | *Lactob acillus sakei* NK158 | + | + | ++ | - | + | + | + |
| 9 | *Lactob acillus paracas ei* NK159 | - | + | ++ | - | - | + | - |
| 10 | *Lactob acillus helvetic us* NK160 | + | + | + | - | - | - | - |
| 11 | *Lactob acillus bulgari cus* NK161 | + | + | + | + | + | + | + |
| 12 | *Lactob acillus pentosu* s NK162 | - | ++ | ++ | - | + | + | + |
| 13 | *Lactob acillus brevis* NK163 | + | + | ++ | - | + | - | + |
| 14 | *Lactob acillus brevis* NK164 | + | + | + | + | + | - | + + |
| 15 | *Lactob acillus reuteri* NK165 | + | ++ | + | + | + | - | + |
| 16 | *Lactob acillus reuteri* NK166 | + | + | + | - | + | + | + |
| 17 | *Lactob acillus johnson ii* NK167 | + | + | + | - | + | + | + |
| 18 | *Lactob acillus johnson ii* NK168 | + | ++ | ++ | + | + | - | + |
| 19 | *Lactob acillus rhamno sus* NK169 | + | + | + | + | + | - | + |
| 20 | *Lactob acillus rhamno sus* NK170 | - | + | + | - | + | - | + + |
| 21 | *Bifidob acterium adolesc entis* NK171 | - | - | + | + | + | - | + |
| 22 | *Bifidob acteriu* m *adolesc entis* NK 172 | - | - | + | + | + | - | + |
| 23 | *Bifidob acteriu* m *longum* NK173 | +++ | + | + | + | + | - | + |
| 24 | *Bifidob acteriu* m *longum* NK174 | - | + | + | + | + | + | + |
| 25 | *Bifidob acteriu m bifidum* NK175 | ++ | +++ | ++ | ++ | + | - | + + + |
| 26 | *Bifidob acteriu m bifidum* NK176 | - | + | + | + | + | + | + |
| 27 | *Bifidob acteriu m breve* NK177 | + | ++ | + | + | + | - | + + |
| 28 | *Bifidob acteriu m breve* NK178 | + | + | ++ | + | + | - | + |
| 29 | *Bifidob acteriu* m *animali* s NK 179 | ++ | ++ | ++ | + | + | - | + |
| 30 | *Bifidob acteriu* m *animali* s NK 180 | + | + | ++ | - | + | - | + |

| | | | | | | | | |
|---|---|---|---|---|---|---|---|---|
| ^{∗} Final treated concentration of lactic acid bacteria: 1 × 10⁵ CFU/ml^{∗} +++, > 80%; ++, > 40 -80%; +, > 1 - 40%; -, < 0 - -40%; --, < -40%. | | | | | | | | |

As shown in Table 3, *Lactobacillus plantarum* NK151 and *Bifidobacterium bifidum* NK175 showed excellent IL-10 expression inducing activity compared to TNF-α expression. In addition, *Bifidobacterium longum* NK173 was also confirmed to have excellent IL-10 expression inducing activity compared to the TNF-α expression. From these results, it was confirmed that the strains may exhibit excellent anti-inflammatory activity.

In addition, in order to consider the possibility of normalizing intestinal microbiota, even in a result of confirming the *Proteobacteria* growth inhibitory activity, the above-mentioned strains also showed excellent *Proteobacteria* growth inhibitory activity.

### Example 3. Deposit of strains and confirmation of physiological characteristics

### 3-1. Confirmation of novel strains and confirmation of 16s rDNA

It was confirmed that isolated strains of *Lactobacillus plantarum* NK151, *Bifidobacterium bifidum* NK175 and *Bifidobacterium longum* NK173 were all Gram-positive bacteria, and bacilli.

As a result of confirming 16S rDNA, it was found that 16S rDNA of *Lactobacillus plantarum* NK151 had a base sequence represented by SEQ ID NO: 1. As a result of homology analysis of 16S rDNA of *Lactobacillus plantarum* NK151 using a BLAST program of the gene bank, the known *Lactobacillus plantarum* strain was not searched, and had 99% homology with a 16S rDNA sequence of the known *Lactobacillus plantarum* strain. The novel strain was named as *Lactobacillus plantarum* NK151, patent-deposited with the Korean Culture Center of Microorganisms (address: Yurim Building, 45, Hongjenae 2-ga-gil, Seodaemun-gu, Seoul, Korea), an authorized depository institution on September 10, 2020, and given the accession number of KCCM12783P.

The 16S rDNA of *Bifidobacterium bifidum* NK175 was found to have a base sequence represented by SEQ ID NO: 2. As a result of homology analysis of 16S rDNA of *Bifidobacterium bifidum* NK175 using a BLAST program of the gene bank, the known *Bifidobacterium bifidum* strain was not searched, and had 99% homology with a 16S rDNA sequence of the known *Bifidobacterium bifidum* strain. The novel strain was named as *Bifidobacterium bifidum* NK175, patent-deposited with the Korean Culture Center of Microorganisms (address: Yurim Building, 45, Hongjenae 2-ga-gil, Seodaemun-gu, Seoul, Korea), an authorized depository institution on September 10, 2020, and given the accession number of KCCM12784P.

In addition, the 16S rDNA of *Bifidobacterium longum* NK173 was found to have a base sequence represented by SEQ ID NO: 3. As a result of homology analysis of 16S rDNA *of Bifidobacterium longum* NK173 using a BLAST program of the gene bank, the known *Bifidobacterium longum* strain was not searched, and had 99% homology with a 16S rDNA sequence of the known *Bifidobacterium longum* strain. The novel strain was named as *Bifidobacterium bifidum* NK175, patent-deposited with the Korean Culture Center of Microorganisms (address: Yurim Building, 45, Hongjenae 2-ga-gil, Seodaemun-gu, Seoul, Korea), an authorized depository institution on September 6, 2021, and given the accession number of KCCM13046P.

### 3-2. Biochemical identification using API kit

The carbon sources among physiological characteristics of *Lactobacillus plantarum* NK151, *Bifidobacterium bifidum* NK175 and *Bifidobacterium longum* NK173 were analyzed by a sugar fermentation test with API 50CH Kit and API 20A Kit (Manufacturer: BioMerieux's, USA), respectively, and the results thereof were shown in Tables 4 to 6 below, respectively.

**[Table 4]**

| Carbon source | NK151 | Carbon source | NK151 |
|---|---|---|---|
| - | - | Salicin | + |
| Glycerol | + | Cellobiose | + |
| Erythritol | - | Maltose | + |
| D-arabinose | - | Lactose | + |
| L-arabinose | + | Melibiose | + |
| D-ribose | + | Sucrose | + |
| D-xylose | - | Trehalose | + |
| L-xylose | - | Inulin | - |
| D-adonitol | - | Melezitose | + |
| Methyl-β-D-xylopyranoside | - | Raffinose | + |
| D-galactose | + | Starch | - |
| D-glucose | + | Glycogen | - |
| D-fructose | + | Xylitol | - |
| D-mannose | + | Gentiobiose | + |
| L-sorbose | - | D-turanose | + |
| L-rhamnose | - | D-lyxose | - |
| Dulcitol | - | D-tagatose | - |
| Inositol | - | D-fucose | - |
| Mannitol | + | L-fucose | - |
| Sorbitol | - | D-arabitol | - |
| α-methyl-D-mannoside | + | L-arabitol | - |
| α-methly-D-glucoside | - | Gluconate | + |
| N-acetyl-glucosamine | + | 2-keto-gluconate | - |
| Amygdalin | + | 5-keto-gluconate | - |
| Arbutin | + | Esculin | + |

**[Table 5]**

| Carbon source | NK175 | Carbon source | NK175 |
|---|---|---|---|
| L-tryptophan | - | Esculin | + |
| Urea | - | Glycerol | - |
| D-glucose | + | D-cellobiose | + |
| D-mannitol | - | D-mannose | + |
| D-lactose | + | D-melezitose | - |
| D-sucrose | - | D-raffinose | + |
| D-maltose | + | D-sorbitol | - |
| Salicin | - | D-rhamnose | - |
| D-xylose | + | D-trehalose | - |
| L-arabinose | - | CAT | - |
| Gelatin | - | Spor | - |
| Gram | + | Cocc | - |

**[Table 6]**

| Carbon source | NK173 | Carbon source | NK173 |
|---|---|---|---|
| L-tryptophan | - | Esculin | + |
| Urea | - | Glycerol | - |
| D-glucose | + | D-cellobiose | - |
| D-mannitol | + | D-mannose | + |
| D-lactose | - | D-melezitose | - |
| D-sucrose | - | D-raffinose | - |
| D-maltose | + | D-sorbitol | - |
| Salicin | - | D-rhamnose | - |
| D-xylose | + | D-trehalose | - |
| L-arabinose | + | CAT | - |
| Gelatin | + | Spor | - |
| Gram | + | Cocc | - |

In Tables 4 to 6, "+" indicated a case in which carbon source availability was positive, and "-" indicated a case in which carbon source availability was negative. As shown in Tables 4 to 6, it was confirmed that the biochemical characteristics of each strain were different and not the same.

### Example 4: Preparation of dead cells and lysates

### Example 4-1. Preparation of dead cells

*Lactobacillus plantarum* NK151, *Bifidobacterium bifidum* NK175, and *Bifidobacterium longum* NK173 were subjected to heat treatment at 90°C for 10 minutes three times under heat treatment conditions to prepare dead cells.

### Example 4-2. Preparation of lysates (soluble and insoluble substances)

Viable cells obtained by centrifuging *Lactobacillus plantarum* NK151, *Bifidobacterium bifidum* NK175 and *Bifidobacterium longum* NK173 were suspended by sterile distillation to be 1 × 10⁶ CFU/mL, sonicated at 4°C (1 minute treatment, 1 minute break repeated 5 times), and centrifuged (10,000 g, 4°C, 15 minutes) to obtain a supernatant (soluble substance) and a precipitate (insoluble substance). The supernatant and the precipitate were dried and used.

### Example 5. Confirmation of activities of dead cells and lysates

Anti-inflammatory activity in macrophages was confirmed as in Example 2 using the dead cells and the lysates prepared in Example 4.

As a result, both the dead cells prepared through heat treatment (90°C, 10 minutes, 3 times treatment) and the lysates lysed by sonication (1 minute, 10 times) showed IL-10 expression inducing activity compared to TNF-a expression similar to a case when the viable cells were treated.

### Example 6. Confirmation of treatment and alleviation effects in eye disease model

### 6-1. Production of experimental animals for eye diseases

8-week-old BALB/c mice (Samtako, Korea) were purchased, anesthetized with 2% isoflurane to remove the lacrimal gland after one week acclimatization, and while maintaining with 1% isoflurane, hair was removed in the middle between the eyes and ears of the mouse, the surgical site was disinfected, and then about 1 cm of the skin were incised. After the exorbital lacrimal gland was removed from the incision site, the skin was sutured, and the cornea was treated with atropine sulfate for 5 days. The next day, a phenol-red thread tear test strip (FCI Opthalmics Zone Quick, Japan) was in contact with the ocular surface at the outer end of the eyelid and after 30 seconds, the length of a color change of the test strip due to tears was measured, and mice with significantly reduced lacrimal secretion compared to a normal control group (sham) were used in the experiment. There were 8 mice per group. The mice were divided into the following groups:
1) a normal control group (NC),
2) a sham group (sham) in which only surgery was performed and the lacrimal gland was not removed,
3) a group (DE) in which the lacrimal gland was removed and physiological saline was administered,
4) a group (LP) in which the lacrimal gland was removed and *Lactobacillus plantarum* NK151 was administered,
6) a group (BB) in which the lacrimal gland was removed and *Bifidobacterium bifidum* NK175 was administered,
6) a group (BL) in which the lacrimal gland was removed and *Bifidobacterium longum* NK173 was administered,
7) a group (LB1) in which the lacrimal gland was removed and *Lactobacillus plantarum* NK151 and *Bifidobacterium bifidum* NK175 were mixed at 1 : 1 and administered,
8) a group (LB2) in which the lacrimal gland was removed and *Lactobacillus plantarum* NK151 and *Bifidobacterium bifidum* NK175 were mixed at 4 : 1 and administered, and
9) a group (LB3) in which *Lactobacillus plantarum* NK151 and *Bifidobacterium longum* NK173 were mixed at 4 : 1 and administered.

### 6-2. Measurement of lacrimal secretion

For the lacrimal secretion, vehicle (1% maltose solution) was administered in the normal control group, the sham group, and the DE group, and lactic acid bacteria were administered in the LP, BB, BL, LB1, LB2, and LB3 groups as a separate experiment set for 10 days at 5 × 10⁸ CFU/mouse/day, respectively (in the case of mixed administration, the above-mentioned dose was mixed and administered according to the CFU-based ratio).

The lacrimal secretion was measured by contacting the phenol-red thread tear test strip (FCI Opthalmics Zone Quick, Japan) with the ocular surface at the outer end of the eyelid, and then measuring the length of the color change of the test strip by tears after 30 seconds.

The results were shown in Table 7.

Table 7 below showed the results of administration of 5 × 10⁸ CFU/mouse/day for 10 days.

**[Table 7]**

| Experimental group | Lacrimal secretion (mm) |
|---|---|
| Normal control group | 4.8 |
| SH group | 4.9 |
| DE group | 1.2 |
| LP group | 2.1 |
| BB group | 1.8 |
| BL group | 1.6 |
| LB1 group | 2.0 |
| LB2 group | 2.1 |
| LB3 group | 1.9 |

As may be seen in Table 7, the lacrimal secretion of the normal control group, the sham group, and the DE group, which were administered with the vehicle solution, was 4.8, 4.9, and 1.2 mm, respectively, and after 10 days, the lacrimal secretion in mice induced with eye diseases was significantly reduced compared to the normal control group. The LP group administered *with Lactobacillus plantarum* NK 151, the BB group administered with *Bifidobacterium bifidum* NK175, the LB1 group administered with 1 : 1 mixed bacteria, and both the LB2 and LB3 groups administered with 4 : 1 mixed bacteria, the lacrimal secretion was significantly increased according to the administration of lactic acid bacteria.

As these results, the lacrimal secretion was increased even in mice administered with 1 × 10⁸ CFU/mouse/day.

It was confirmed that the increase in lacrimal secretion was higher when the strains was used together, and particularly, in the case of *Bifidobacterium bifidum* NK175 and *Bifidobacterium longum* NK173, it was confirmed that the lacrimal secretion was significantly increased in the LB1 to LB3 groups mixed with *Lactobacillus plantarum* NK151 as compared to when administered alone.

The result showed that *Lactobacillus plantarum* NK151, *Bifidobacterium bifidum* NK175 and *Bifidobacterium longum* NK173 or any mixture thereof increased the lacrimal secretion to be effective for alleviation of eye diseases.

### 6-3. Confirmation of corneal fluorescein staining

The mice in 6-1 were anesthetized, and then the degree of corneal damage was observed using a fluorescent dye (Fluorescein sodium salt). Specifically, a 2.5% fluorescein solution was dropped into the eyeball of the experimental animal, and then photographed with a biomicroscope under cobalt blue light, and the cornea was divided into upper, middle, and lower areas, and the most severe corneitis was evaluated as 3 points in each area.

**[Table 8]**

| Experimental group | Corneal fluorescein staining score |
|---|---|
| Normal control group | 0.4 |
| SH group | 0.5 |
| DE group | 4.8 |
| LP group | 1.4 |
| BB group | 1.9 |
| BL group | 1.9 |
| LB1 group | 1.5 |
| LB2 group | 1.4 |
| LB3 group | 1.9 |

As shown in Table 8, in the DE group treated with atropine after removing the lacrimal gland, the cornea was significantly damaged compared to the normal control group, but in the LP group, BB group, BL group, LB1 group, LB2 group and LB3 group treated with *Lactobacillus plantarum* NK151, *Bifidobacterium bifidum* NK175 and/or *Bifidobacterium longum* NK173, the corneal damage was significantly alleviated.

### 6-4. Measurement of expression level of corneal cytokines

50 mg of the cornea was homogenized by adding 150 µl of an RIPA buffer containing protease inhibitor cocktail. Thereafter, a supernatant was obtained by centrifugation at 4°C and 10,000 g for 15 minutes, and 50 µl of the supernatant was put into a 96-well and the expression levels of TNF-α, IL-1β, and IL-10 were measured using ELISA plate kits (Pierce Biotechology, Inc., Rockford, IL, USA).

First, 50 µl of the supernatant was put into a 96-well plate coated with TNF-α, IL-1β, and IL-10 antibodies, respectively, reacted at room temperature for 2 hours, and then washed with phosphate buffered saline-Tween (PBS-Tween), and added with a color developing agent and developed for 20 minutes, and the absorbance was measured and calculated at a wavelength of 450 nm.

**[Table 9]**

| Experimental group | TNF-α (pg/mg) | IL-1β (pg/mg) | IL-10 (pg/mg) |
|---|---|---|---|
| Normal control group | 1.2 | 10.2 | 18.2 |
| SH group | 1.8 | 14.3 | 17.4 |
| DE group | 15.6 | 46.5 | 7.8 |
| LP group | 4.8 | 21.9 | 13.5 |
| BB group | 5.9 | 31.5 | 11.0 |
| BL group | 6.9 | 33.2 | 14.5 |
| LB1 group | 5.4 | 22.6 | 12.5 |
| LB2 group | 5.1 | 21.3 | 12.4 |
| LB3 group | 1.6 | 24.5 | 13.0 |

As shown in Table 9, in the DE group treated with atropine after removing the lacrimal gland, the expression levels of the inflammatory cytokines TNF-α and IL-1β were significantly increased compared to the normal control group, and the expression level of the anti-inflammatory cytokine IL-10 was decreased. Meanwhile, in all of the LP group, BB group, BL group, LB1 group, LB2 group and LB3 group treated with *Lactobacillus plantarum* NK151, *Bifidobacterium bifidum* NK175 and/or *Bifidobacterium longum* NK173, not only the corneal damage was significantly lowered, but also the expression level of IL-10 was increased and the expression levels of the inflammatory cytokines TNF-α and IL-1β were decreased. In particular, it was confirmed that the expression control effect of inflammatory and anti-inflammatory cytokines was excellent in the LP group, LB2 group and LB3 group, and thus, the results suggested that *Lactobacillus plantarum* NK151, or a strain mixed with *Bifidobacterium bifidum* NK175 or *Bifidobacterium longum* NK173 was effective lactic acid bacteria in alleviation of eye diseases, particularly alleviation of inflammation in the eyes.

### 6-5. Measurement of amounts of malondialdehyde (MDA), glutathione (GSH), and nitric oxide (NO) in the retina

The following experiments were performed to measure anti-oxidant-related factors and NO, which may be considered important in eye diseases, and to confirm changes in inflammation level in the retina.

The retina was separated from the eyeball excised with fine scissors. The excised retina (1 mg) was put into 0.2 mL of cold PBS and homogenized with a homogenizer in a cold room.

MDA measurement: 0.6 ml of 1% phosphoric acid and 0.2 ml of 0.6% thiobarbituric acid were added to 50 µl of a retinal homogenate, and then boiled at 95°C for 45 minutes and cooled, and then added with 0.8 ml of n-butanol and centrifuged at 3,000 g for 20 minutes. After centrifugation, a supernatant was taken and absorbance was measured at 535 nm. For the MDA standard curve, 535 nm absorbance was measured using 1,1,3,3-tetraethoxypropane (malondialdehyde, MDA).

NO measurement: NO in 50 µl of the retinal homogenate was measured using a Nitrate/Nitrite Colormetric Assay Kit (Caymanchem, 780001).

GSH measurement: 600 µl of a solution R3, 50 µl of a solution R1, and 50 µl of a solution R2, which were reagents in a GSH determination kit, were sequentially mixed in 50 µl of the retinal homogenate and vortexed, and then reacted for 10 minutes in the dark at 25°C, and 405 nm absorbance was measured.

TNF-α measurement: TNF-α was measured using ELISA plate kits (Pierce Biotechology, Inc., Rockford, IL, USA).

**[Table 10]**

| Experimental group | MDA (nmol/g) | Glutathione (µmol/g) | NO^{∗} | TNF-α (pg/mg) |
|---|---|---|---|---|
| Normal control group | 31.9 | 2721.7 | 100.0 | 0.8 |
| SH group | 32.5 | 2798.5 | 108.1 | 0.9 |
| DE group | 42.3 | 3105.2 | 133.2 | 1.6 |
| LP group | 33.8 | 2795.6 | 112.8 | 1.1 |
| BB group | 35.4 | 2880.3 | 118.9 | 1.3 |
| BL group | 38.5 | 2987.0 | 123.5 | 1.4 |
| LB1 group | 34.1 | 2796.9 | 111.4 | 1.1 |
| LB2 group | 34.9 | 2845.6 | 117.0 | 1.2 |
| LB3 group | 36.4 | 2987.5 | 125.3 | 1.3 |

| | | | | |
|---|---|---|---|---|
| ^{∗} Normal control group was represented as 100% | | | | |

As shown in Table 10, in the DE group treated with atropine after removing the lacrimal gland, the concentrations of malondialdehyde (MDA), glutathione (GSH), nitric oxide, and TNF-α in the retina were increased compared to the normal control group.

Meanwhile, in all of the LP group, BB group, BL group, LB1 group, LB2 group and LB3 group treated with *Lactobacillus plantarum* NK151, *Bifidobacterium bifidum* NK175 and/or *Bifidobacterium longum* NK173, the concentrations of malondialdehyde (MDA), glutathione (GSH), nitric oxide, and TNF-α in the retina were lowered. In particular, in the LP group, LB2 group, and LB3 group, it was confirmed that the production inhibitory control effect of malondialdehyde (MDA), glutathione (GSH), and nitric oxide, which were production indicators of radicals that induced macular degeneration, was excellent.

The results suggested that *Lactobacillus plantarum* NK151, or a strain mixed with *Bifidobacterium bifidum* NK175 or *Bifidobacterium longum* NK173 is effective lactic acid bacteria in inhibition and alleviation of the macular degeneration of the eyeball.

### 6-6. Measurement of blood concentrations of corticosterone, creatinine, and lactate and lactate dehydrogenase activity

When fatigue including eye fatigue accumulated in the body, a lot of fatigue-related substances (e.g., lactic acid, creatinine, etc.) were accumulated, and a lot of stress-related hormones (e.g., corticosterone, etc.) were accumulated. Accordingly, the alleviation effect on the fatigue phenomenon was confirmed by confirming an expression change of the factors.

The blood was collected from the mouse model of 6-1 above, put into a test tube with EDTA, treated with EDTA at 3000 rpm and 4°C for 15 minutes, and plasma was isolated. Concentrations of lactate and creatinine and lactate dehydrogenase activity in the plasma were measured using lactate, creatinine, and lactate dehydrogenase assay kits from Abcam (Cambridge, MA, USA). In addition, the concentration of corticosterone in the plasma was measured using an ELISA assay kit from Elabscience (Hebei, China).

**[Table 11]**

| Experimental group | Lactic acid (mM) | LDH (U/L) | Creatinine (mg/dL) | Corticosterone (ng/mL) |
|---|---|---|---|---|
| Normal control group | 5.4 | 2472.1 | 0.11 | 38.5 |
| SH group | 5.9 | 2580.3. | 0.12 | 45.3 |
| DE group | 8.8 | 3171.5 | 1.96 | 95.4 |
| LP group | 5.8 | 2578.9 | 1.28 | 44.9 |
| BB group | 6.5 | 2758.3 | 1.32 | 52.7 |
| BL group | 7.2 | 2812.0 | 1.54 | 57.2 |
| LB1 group | 5.8 | 2567.2 | 1.29 | 44.6 |
| LB2 group | 5.9 | 2612.5 | 1.31 | 43.8 |
| LB3 group | 6.9 | 2768.4 | 1.44 | 45.1 |

As shown in Table 11, in the DE group treated with atropine after removing the lacrimal gland, the plasma concentrations of lactate, creatinine, and corticosterone and the LDH activity were increased compared to the normal control group. Meanwhile, in all of the LP group, BB group, BL group, LB1 group, LB2 group and LB3 group treated with *Lactobacillus plantarum* NK151, *Bifidobacterium bifidum* NK175 and/or *Bifidobacterium longum* NK173, the plasma concentrations of lactate, creatinine, and corticosterone and the LDH activity were lowered. In particular, it was confirmed that the inhibitory control effects of the concentrations of lactate, creatinine, and corticosterone and the LDH activity as indicators of fatigue were excellent in the LP group, LB2 group and LB3 group. Thus, the results suggested that *Lactobacillus plantarum* NK151, or a strain mixed with *Bifidobacterium bifidum* NK175 or *Bifidobacterium longum* NK173 was effective lactic acid bacteria in inhibition and alleviation of fatigue caused by dry eyes, surgery, and stress.

### Example 7. Preparation of depression, anxiety, fatigue, and colitis disease models and confirmation of treatment efficacy

### 7-1. Preparation of restraint stress animal model

A restraint stress animal model was prepared to induce mental disorders such as depression and stress. First, C57BL/6 male mice (5 weeks old, 19 to 21 g) were acclimated in a laboratory for 1 week by 8 mice per group. One group was a normal control group and other groups were experimental groups. In the experimental groups, the mice were fixed to a 3 × 10 cm cylindrical restraint stress device so as not to move. Specifically, the immobilization stress (IS) was repeated 5 times by placing the head upright every 2 hours once every 2 days, and lactic acid bacteria and complexes thereof (administered with 5 × 10⁸ CFU/mouse/day and used the same as the previous strain ratio) were administered once daily for 5 days from 24 hours after the last restraint stress. Physiological saline was administered to the normal control group (NC).

As behavioral experiments to confirm the efficacy of the isolated lactic acid bacteria in alleviating depression/anxiety and fatigue, an elevated plus maze test, a tail suspension test, and a forced swimming test were conducted, and in the hippocampus, IL-6 and BDNF were measured by an ELISA kit, in the blood, corticosterone and IL-6 were measured by an ELISA kit or assay kit, and in the colon, IL-1β, IL-6, IL-10, and MPO were measured by an ELISA kit.

### 7-2. Preparation of depression/cognitive disorder/colitis disease model

A fecal transplant animal model was prepared to induce mental disorders caused by depression/cognitive disorder/colitis. First, C57BL/6 male mice (5 weeks old, 19 to 21 g) were acclimated in a laboratory for 1 week by 8 mice per group. One group was a normal control group and other groups were experimental groups. In the experimental groups, feces (2 g) of patients (female in 60s) with depression/cognitive disorder/colitis were suspended in physiological saline (18 mL), centrifuged at 500 g for 5 minutes, and a supernatant was separated. This supernatant (0.1 mL) was fecal-transplanted into mice once daily for 5 days. From 24 hours after the last fecal microbiota transplantation (FMT), once daily for 5 days, lactic acid bacteria and complexes thereof (administered with 5 × 10⁸ CFU/mouse/day and used the same as the previous strain ratio) were administered. Physiological saline was administered to the normal control group (NC).

As behavioral experiments to confirm the efficacy of the isolated lactic acid bacteria in alleviating depression/anxiety and fatigue, an elevated plus maze test, a tail suspension test, and a forced swimming test were conducted, and in the hippocampus, IL-6 and BDNF were measured by an ELISA kit, in the blood, the concentrations of corticosterone, IL-6, creatinine, and lactic acid were measured by an ELISA kit or assay kit, and in the colon, IL-1β, IL-6, IL-10, and MPO were measured by an ELISA kit.

### 7-3. Confirmation of alleviation effects on colitis and dementia, anxiety and depression caused thereby

6-week-old male C57BL/6 mice (Samtako, Korea) were purchased and acclimatized for a week in an animal laboratory (humidity 50 ± 10%, temperature 25 ± 2°C, light repeated every 12 hours, air HEPA filtration) and used. As a feed, a standard experimental feed (Samyang, Korea) was used, and drinking water was freely consumed. The experimental animal was lightly anesthetized with ether, and 0.1 ml of TNBS 2.5 mg/5% EtOH was administered into the colon through the anus using a 1 mL syringe with a round tip, and held vertically for 30 seconds. On the other hand, 0.1 ml of physiological saline was orally administered to the normal control group. One group consisted of 6 mice.

A sample (administered with 5 × 10⁸ CFU/mouse/day and used the same as the previous strain ratio) was dissolved in physiological saline once a day for 3 days from the next day and orally administered according to a predetermined dose. On the next day after sample administration was completed, the experimental animals were suffocated with carbon dioxide and the colon was extracted from the appendix to a portion just before the anus. Meanwhile, the results were secured by performing the colon length, the MPO expression, cytokine expression level of the colon, brain-derived neurotrophic factor (BDNF) expression level, Y-maze task, tail suspension test, etc.

### 7-4. Y-maze task

A memory alleviation effect was confirmed through a Y-maze task with respect to the animal models.

Specifically, a Y-maze measurement device extends three arms to form a Y-lettered shape, and each branch had a length of 25 cm, a height of 14 cm, and a width of 5 cm, and was positioned at the same angle. The head of the experimental animal faced the end of one arm of the Y-maze and the experimental animal freely moved around the arm for 8 minutes. The movement of the animal was recorded, and the animal entered the arm up to its hind feet, which was regarded as arm entry. The movement of the animal was represented by the alternation, and the alternation was defined as one alternating when the animal passed through three arms in succession. The spontaneous alternating amount was represented as a percentage of an actual alternation and a maximum possible alternation (that is, the value obtained by subtracting 2 from the total alternation).

### 7-5. Elevated plus maze (EPM) test

The test was performed on the animal models. In a room with a video camera installed above at a brightness of 20 Lux, in an elevated plus maze test device [two open arms (30 × 7 cm) and two enclosed arms (30 × 7 cm) with walls of 20 cm high were placed 50 cm high from the floor, and a black plexiglass unit extending by 7 cm from a central platform], the mouse was placed to face the open arm with its head in the center, and the time and number of times spent in the open and closed arms for 5 minutes were measured. The time spent in open arms (OT) during the entire experiment time was calculated as [time spent in open arms / (time spent in open arms + time spent in closed arms)] × 100.

### 7-6. Tail Suspension Test (TST)

The test was performed on the animal models. A fixing device was mounted to about 1 cm from the tip of the mouse's tail to a container with a diameter of 35 cm and a height of 50 cm, and then the mouse was suspended at a distance of 50 cm from the ground. The immobility time of the experimental animals was measured for a total of 6 minutes.

### 7-7. Forced Swimming Test (FST)

The test was performed on the animal models. A water tank with a height of 40 cm and a diameter of 20 cm was filled with water at a temperature of 25 ± 1°C to a height of 30 cm. The disease model mice prepared in Experimental Example 5 were placed in the water tank one by one and first 2 minutes out of a total of 6 minutes were not measured as adaptation time, and the immobility time of the experimental animals for the last 4 minutes was measured (represented by percentage). The immobility refers to a state of standing upright and floating motionless, with only minimal movement to expose only the head above the water.

### 7-8. Measurement of colon length

The abdomen was dissected to separate the colon and the length of the colon was measured.

### 7-9. Measurement of myeloperoxidase (MPO) activity

100 mg of the colon tissue was added with 200 µl of 10 mM potassium phosphate buffer (pH 7.0) containing 0.5% hexadecyl trimethyl ammonium bromide, and homogenized. Then, a supernatant was obtained by centrifugation for 10 minutes under conditions of 4°C and 10,000 g. The supernatant was used as a crude enzyme solution. 50 µl of the crude enzyme solution was added to 0.95 µl of a reaction solution (containing 1.6 mM tetramethyl benzidine and 0.1 mM H₂O₂), and reacted at 37°C, and the absorbance was measured over time at 650 nm. The activity of the myeloperoxidase was calculated as 1 unit based on 1 µmol/ml of H₂O₂ generated as a reactant.

### 7-10. Measurement of expression level of cytokines in colon

100 mg of colon tissue was added with 250 µl of an RIPA buffer containing a protease inhibitor cocktail and homogenized. Thereafter, a supernatant was obtained by centrifugation at 4°C and 10,000 g for 15 minutes, and 50 µl of the supernatant was put into a 96-well and the expression levels of TNF-α, IL-1β, and IL-6 were measured using ELISA plate kits (Pierce Biotechnology, Inc., Rockford, IL, USA).

First, 50 µl of the supernatant was put into a 96-well plate coated with TNF-α, IL-1β, and IL-6 antibodies, respectively, reacted at room temperature for 2 hours, and then washed with phosphate buffered saline-Tween (PBS-Tween), and added with a color developing agent and developed for 20 minutes, and the absorbance was measured and calculated at a wavelength of 450 nm.

### 7-11. Confirmation of effect in restraint stress model

**[Table 12]**

| | EPM | TST | FST |
|---|---|---|---|
| NC group | 19.4 | 32.3 | 42.1 |
| IS group | 6.5 | 45.5 | 64.8 |
| LP group | 12.7 | 35.2 | 50.7 |
| BB group | 14.3 | 34 | 43.3 |
| BL group | 11.3 | 31.5 | 45.5 |
| LB1 group | 15.3 | 33.6 | 43.3 |
| LB2 group | 15.61 | 33.2 | 44.4 |
| LB3 group | 15.8 | 33.8 | 43.6 |

It was confirmed that in the group treated with restraint stress (IS), the anxiety and depressive behavior were increased significantly in EPM, TST, and FST compared to the normal control group, but in the LP, BB, BL, LB1, LB2, and LB3 groups, the anxiety and depressive behavior increased by restraint stress were significantly suppressed. That is, it was confirmed that the anxiety and depressive behavior caused by restraint stress were excellently alleviated in all groups administered with *Lactobacillus plantarum* NK151, *Bifidobacterium bifidum* NK175, and *Bifidobacterium longum* NK173, and the alleviation effect was also confirmed even by co-administration.

**[Table 13]**

| | Hippocampus | | Blood | | Colon | | |
|---|---|---|---|---|---|---|---|
| | IL-6 (pg/mg) | BDNF (ng/mg) | Corticost erone (ng/mL) | IL-6 (pg/mL) | MPO (ng/mg) | IL-1β (pg/mg) | IL-10 (pg/mg) |
| NC group | 38.2 | 1.2 | 42.1 | 7.2 | 0.32 | 30.2 | 89.0 |
| IS group | 44.6 | 0.6 | 81.8 | 11.5 | 0.72 | 48.5 | 42.2 |
| LP group | 41.2 | 0.9 | 51.3 | 8.5 | 0.46 | 42.4 | 69.3 |
| BB group | 39.4 | 1.0 | 52.2 | 8.4 | 0.42 | 35.6 | 72.2 |
| BL group | 41.5 | 1.0 | 54.5 | 8.5 | 0.45 | 34.9 | 73.5 |
| LB1 group | 40.2 | 1.1 | 55.7 | 8.2 | 0.43 | 33.8 | 77.2 |
| LB2 group | 40.5 | 1.0 | 55.5 | 8.2 | 0.44 | 33.1 | 78.5 |
| LB3 group | 40.1 | 1.1 | 53.5 | 8.3 | 0.43 | 33.7 | 76.9 |

In the group treated with restraint stress (IS), compared to the normal control group, IL-6 in the hippocampus, corticosterone and IL-6 in the blood, and MPO and IL-1β in the colon were significantly increased, but BDNF in the hippocampus and IL-10 in the colon were significantly decreased. However, in the *Lactobacillus plantarum* NK151, *Bifidobacterium bifidum* NK175, and *Bifidobacterium longum* NK173, IL-6 in the hippocampus, corticosterone and IL-6 in the blood, and MPO and IL-1β in the colon which have been increased by the stress and the like were decreased, whereas BDNF which has been decreased in the hippocampus and IL-10 in the colon were increased. In addition, the alleviation effect was excellent even by the co-administration.

### 7-12. Confirmation of effect in fecal transplanted mice

**[Table 14]**

| | EPM | TST | FST | Y-maze |
|---|---|---|---|---|
| NC group | 24.5 | 30.0 | 38.6 | 72.3 |
| FMT group | 12.3 | 43.6 | 72.0 | 52.4 |
| LP group | 16.4 | 33.4 | 44.8 | 63.4 |
| BB group | 19.1 | 32.0 | 45.0 | 66.1 |
| BL group | 18.7 | 32.4 | 45.3 | 65.9 |
| LB1 group | 17.9 | 32.3 | 45.0 | 68.7 |
| LB2 group | 19.3 | 32.7 | 43.7 | 68.1 |
| LB3 group | 19.5 | 31.9 | 42.5 | 68.7 |

In the group subjected to Fecal Microbiota Transplantation (FMT), compared to the normal control group, in EPM, TST, and FST, the anxiety and depressive behavior were increased significantly and cognitive impairment was shown in the Y-maze. However, in the LP group, BB group, BL group, LB1 group, LB2 group, and LB3 group, it was confirmed that the increased anxiety and depressive behaviors by the FMT were significantly suppressed and the impaired cognitive ability was significantly alleviated. That is, it was confirmed that the anxiety and depressive behavior caused by the FMT were excellently alleviated in all groups administered with *Lactobacillus plantarum* NK151, *Bifidobacterium bifidum* NK175, and *Bifidobacterium longum* NK173, and the alleviation effect was also confirmed even by co-administration.

**[Table 15]**

| | Hippocampus | | Blood | | | | Colon | | |
|---|---|---|---|---|---|---|---|---|---|
| | IL-6 Pg/mL | BDNF (ng/mg) | Corti coste rone (ng/ mL) | IL-6 | Creati nine (mg/d L) | Lactate (mM) | MPO (ng/ mg) | IL-1β (pg/ mg) | IL-10 (pg/ mg) |
| NC group | 35.9 | 1.4 | 37.6 | 5.8 | 0.09 | 5.8 | 0.27 | 21.1 | 74.5 |
| FMT group | 47.0 | 0.6 | 78.5 | 10.4 | 0.14 | 9.7 | 0.63 | 43.8 | 37.5 |
| LP group | 40.9 | 1.1 | 49.5 | 7.8 | 0.12 | 8.2 | 0.49 | 35.5 | 59.4 |
| BB group | 38.5 | 0.9 | 48.5 | 7.2 | 0.11 | 7.8 | 0.4 | 31.8 | 55.9 |
| BL group | 39.7 | 0.8 | 49.3 | 6.9 | 0.12 | 7.7 | 0.43 | 29.6 | 54.3 |
| LB1 group | 38.9 | 0.9 | 47.5 | 7.1 | 0.11 | 8.4 | 0.38 | 30.3 | 53.2 |
| LB2 group | 38.6 | 0.7 | 48.5 | 7.2 | 0.11 | 8.2 | 0.39 | 29.8 | 50.6 |
| LB3 group | 38.2 | 0.8 | 49.0 | 6.9 | 0.11 | 7.9 | 0.36 | 28.5 | 48.4 |

In the group subjected to the FMT, compared to the normal control group, IL-6 in the hippocampus, corticosterone, IL-6, creatinine and lactic acid in the blood, and MPO and IL-1β in the colon were significantly increased, but BDNF in the hippocampus and IL-10 in the colon were decreased. However, in the groups administered with *Lactobacillus plantarum* NK151, *Bifidobacterium bifidum* NK175, and *Bifidobacterium longum* NK173, the expression levels of these disease-related factors were controlled. In addition, the alleviation effect was excellent even by the co-administration.

### 7-13. Confirmation of therapeutic effect in TNBS-induced depression and cognitive impairment mouse model

First of all, in the LP group, BB group, BL group, LB1 group, LB2 group, and LB3 group, it was confirmed that the length of the colon shortened by TNBS treatment was significantly restored. In addition, it was confirmed that MPO activity was significantly alleviated and the expression levels of TNF-α, IL-1β, and IL-6 were significantly inhibited, while the expression level of IL-10 decreased by TNBS was induced.

In addition, the memory alleviation effect was confirmed through the Y-maze test, and the anxiety/depression alleviation effect was confirmed through the tail suspension test (TST).

**[Table 16]**

| Experimental group | Alteration (%) |
|---|---|
| Normal control group | 68.2 |
| TNBS administered group | 54.5 |
| LP(NK151) group | 65.4 |
| BB(NK175) group | 63.2 |
| BL(NK173) group | 66.5 |
| LB1 group | 65.3 |
| LB2 group | 66.2 |
| LB3 group | 67.2 |

As shown in Table 16, in the TNBS-treated group, the alteration was decreased compared to the normal control group, but in the LP, BB, BL, LB1, LB2, and LP3 groups, the alteration reduced by TNBS was increased, and in the LP group, BL group, LB2 group, and LB3 group, the alteration increase rate was particularly excellent. These results suggested that there were excellent effects on the cognitive alleviation, and the anxiety and depression alleviation of *Lactobacillus plantarum* NK151, *Bifidobacterium bifidum* NK175 and/or *Bifidobacterium longum* NK173. In addition, the results of measuring the expression levels of TNF-α and brain-derived neurotrophic factor (BDNF) in the hippocampus were shown in Table 17.

**[Table 17]**

| Experimental group | TNF-α expression level (pg/mg) | BDNF expression level (pg/mg) |
|---|---|---|
| Normal control group | 3.4 | 2.2 |
| TNBS administered group | 8.5 | 0.9 |
| LP(NK151) group | 4.3 | 1.7 |
| BB(NK175) group | 5.1 | 1.4 |
| BL(NK173) group | 4.4 | 1.5 |
| LB1 group | 4.8 | 1.5 |
| LB2 group | 4.4 | 1.6 |
| LB3 group | 4.2 | 1.6 |

As shown in Table 17, it was confirmed that in the TNBS-treated group, the TNF-α expression level was significantly increased compared to the normal control group, but in the LP group, BB group, BL group, LB1 group, LB2 group and LB3 group, the expression level of TNF-α increased by TNBS was significantly inhibited. Meanwhile, the BDNF expression level decreased by TNBS was induced, and among them, the LP group, LB2 group and LB3 group exhibited excellent TNF-α expression inhibitory and BDNF expression inducing effects. In addition, the tail suspension test (TST) measurement results were shown in Table 18.

**[Table 18]**

| Experimental group | Immobility (%) |
|---|---|
| Normal control group | 23.5 |
| TNBS administered group | 48.4 |
| LP(NK151) group | 27.2 |
| BB(NK175) group | 29.1 |
| BL(NK173) group | 27.8 |
| LB1 group | 27.9 |
| LB2 group | 27.0 |
| LB3 group | 26.9 |

As shown in Table 18, it was confirmed that in the TNBS-treated group, the immobility was significantly increased compared to the normal control group, but in the LP group, BB group, BL group, LB1 group, LB2 group and LB3 group, the immobility increased by TNBS was significantly inhibited. These results showed that *Lactobacillus plantarum* NK151, *Bifidobacterium bifidum* NK175 and/or *Bifidobacterium longum* NK173 of the present invention exhibited a therapeutic effect on inflammatory diseases by controlling the expression of inflammatory cytokines and anti-inflammatory cytokines. In addition, through an anti-oxidant action that may exhibit a therapeutic effect in eye diseases, an action such as inhibition of NO production, a cognitive alleviation effect, alleviation of neuroinflammation, etc., it has been confirmed that there are excellent effects in preventing, alleviating, and treating inflammatory diseases, eye diseases, fatigue, cognitive disorders, and mental disorders. Accordingly, it was confirmed that the strains of the present invention may be used in various applications, such as foods and medicines as well as probiotic products.

The aforementioned description of the present invention is to be exemplified, and it may be understood by those skilled in the art that the technical spirit or required features of the present invention may be easily modified in other detailed forms without changing. Therefore, it should be appreciated that the examples described above are illustrative in all aspects and are not restricted. For example, each component described as a single type may be implemented in a distributed manner, and components described as being distributed may also be implemented in a combined form.

The scope of the present invention is represented by claims to be described below, and it is to be interpreted that the meaning and the scope of the appended claims and all changed or modified forms derived from equivalents thereof come within the scope of the present invention.
Depositary Authority Name: Korean Culture Center of Microorganisms (External)
Accession number: KCCM12783P
Accession Date: 20200910
Depositary Authority Name: Korean Culture Center of Microorganisms (External)
Accession number: KCCM12784P
Accession Date: 20200910
Depositary Authority Name: Korean Culture Center of Microorganisms (External)
Accession number: KCCM13046P
Accession Date: 20210906.

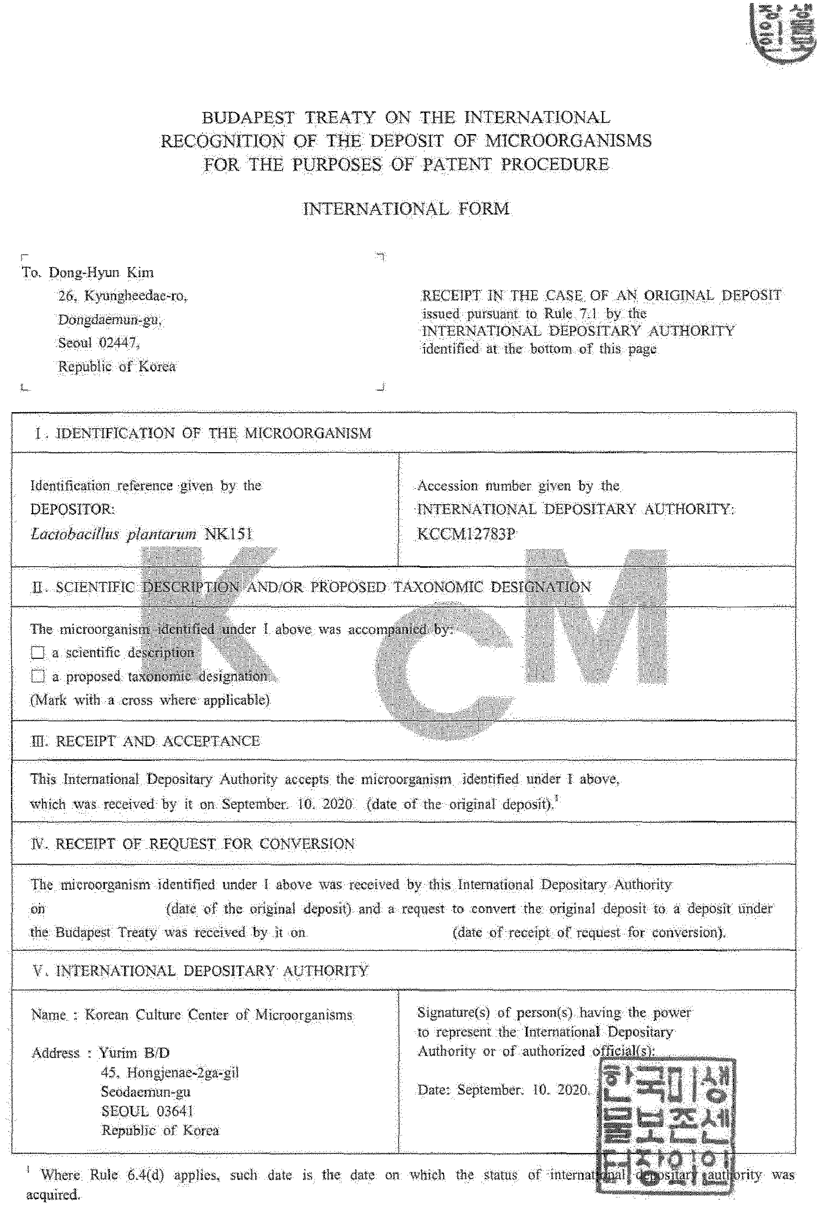
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Dong-Hyun Kim
26, Kyungheedae-ro,
Dongdaemun-gu,
Seoul 02447,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Lactobacillus plantarum NK151 | Accession number given by the
INTERNATIONAL DEPOSITARY AUTHORITY:
KCCM12783P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on September. 10. 2020 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 10. 2020.


¹ Where Rule 6.4(d) applies, such date is the date on which the status of international depositary authority was
acquired.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Dong-Hyun Kim
26, Kyungheedae-ro,
Dongdaemun-gu,
Seoul 02447,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Bifidobacterium bifidum NK175 | Accession number given by the
INTERNATIONAL DEPOSITARY AUTHORITY:
KCCM12784P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 10. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 10. 2020.

¹ Where Rule 6.4(d) applies, such date is the date on which the status of international depositary authority was
acquired.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Kim, Dong-hyun
Kyunghee University,
26, Kyungheedae-ro,
Dongdaemun-gu,
Seoul, Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Bifidobacterium longum NK173 | Accession number given by the
INTERNATIONAL DEPOSITARY AUTHORITY:
KCCM13046P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 06. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 06. 2021.
한국미생물보존센터장인

¹ Where Rule 6.4(d) applies, such date is the date on which the status of international depositary authority was
acquired.

## Claims

1. *Lactobacillus plantarum* NK151 KCCM12783P.

2. The *Lactobacillus plantarum* NK151 KCCM12783P of claim 1, wherein the *Lactobacillus plantarum* NK151 comprises a 16S rDNA base sequence represented by SEQ ID NO: 1.

3. *Bifidobacterium bifidum* NK175 KCCM12784P.

4. The *Bifidobacterium bifidum* NK175 KCCM12784P of claim 2, wherein the *Bifidobacterium bifidum* NK175 comprises a 16S rDNA base sequence represented by SEQ ID NO: 2.

5. *Bifidobacterium longum* NK173 KCCM13046P.

6. The *Bifidobacterium longum* NK173 KCCM13046P of claim 5, wherein the *Bifidobacterium longum* NK173 comprises a 16S rDNA base sequence represented by SEQ ID NO: 3.

7. A pharmaceutical composition for preventing or treating eye diseases, inflammatory diseases, cognitive disorders or mental disorders, comprising *Lactobacillus plantarum* NK151 KCCM12783P, *Bifidobacterium bifidum* NK175 KCCM12784P, *Bifidobacterium longum* NK173 KCCM13046P, or any mixture thereof.

8. The pharmaceutical composition of claim 7, wherein the eye disease is at least one selected from the group consisting of eye fatigue, retinopathy, keratitis, macular degeneration, dry eye syndrome, retinitis pigmentosa, diabetic retinopathy, retinopathy of prematurity, proliferative retinopathy, ischemic retinopathy, choroidal neovascularization, neovascular glaucoma, ischemic optic neuropathy, diabetic macular degeneration, erythema, myopia, and Sjogren's syndrome.

9. The pharmaceutical composition of claim 7, wherein the inflammatory disease is at least one selected from the group consisting of inflammatory bowel disease, arthritis, gout, hepatitis, obesity, gastritis, nephritis, diabetes, tuberculosis, bronchitis, pleurisy, peritonitis, spondylitis, pancreatitis, urethritis, cystitis, vaginitis, arteriosclerosis, sepsis and periodontitis.

10. The pharmaceutical composition of claim 9, wherein the inflammatory bowel disease is at least one selected from the group consisting of ulcerative colitis, Crohn's disease, collagenous colitis, lymphocytic colitis, ischemic colitis, diversion colitis, Behcet's disease, and indeterminate colitis.

11. The pharmaceutical composition of claim 7, wherein the cognitive disorder is at least one selected from the group consisting of Alzheimer's disease, Huntington's disease, vascular dementia, Pick's disease, Parkinson's disease, Creutzfeldt-Jakob disease, and dementia.

12. The pharmaceutical composition of claim 7, wherein the mental disorder is at least one selected from the group consisting of depression anxiety disorder, mood disorder, insomnia, delusional disorder, obsessive-compulsive disorder, migraine, stress, memory disorder, autism, attention-deficit hyperactivity disorder (ADHD), attention-deficit disease (ADD), panic attack and attention disorder.

13. A food composition for preventing or alleviating eye diseases, inflammatory diseases, cognitive disorders or mental disorders, comprising *Lactobacillus plantarum* NK151 KCCM12783P, *Bifidobacterium bifidum* NK175 KCCM12784P, *Bifidobacterium longum* NK173 KCCM13046P, or any mixture thereof.

14. A food composition for alleviating fatigue, comprising *Lactobacillus plantarum* NK 151, *Bifidobacterium bifidum* NK175, *Bifidobacterium longum* NK173, or any mixture thereof.

15. A method for preventing or treating at least one disease selected from the group consisting of eye diseases, cognitive disorders, mental disorders and inflammatory diseases, comprising *Lactobacillus plantarum* NK151, *Bifidobacterium bifidum* NK175, *Bifidobacterium longum* NK173, or any mixture thereof.

16. *Lactobacillus plantarum* NK151, *Bifidobacterium bifidum* NK175, *Bifidobacterium longum* NK173 or any mixture thereof for use in prevention or treatment of at least one disease selected from the group consisting of eye diseases, cognitive disorders, mental disorders and inflammatory diseases.

17. Use of *Lactobacillus plantarum* NK151, *Bifidobacterium bifidum* NK175, *Bifidobacterium longum* NK173 or any mixture thereof in preparation of a medicament for the treatment of at least one disease selected from the group consisting of eye diseases, cognitive disorders, mental disorders and inflammatory diseases.
